# EUROPEAN PATENT SPECIFICATION

(11) **EP 1 155 323 B1**
(45) Date of publication and mention of the grant of the patent: **09.02.2005**
(21) Application number: 99959523.4
(22) Date of filing: 06.12.1999
(51) Int. Cl.: G01N 33/569, G01N 33/577, C12N 5/06, C07K 16/12

(54) **DETECTION METHOD FOR MICROTHRIX PARVICELLA**
NACHWEISVERFAHREN FÜR MICROTHRIX PARVICELLA
PROCEDE DE DETECTION DE MICROTHRIX PARVICELLA

(30) Priority: 05.12.1998 GB 9826758
(43) Date of publication of application: 21.11.2001
(73) Proprietor: Questor Technologies Limited, Belfast BT9 5BN (GB)
(72) Inventor: LARKIN, Michael, James, Belfast BT6 0ND (GB); PATRICK, Sheila, Belfast BT6 0ND (GB); THOMPSON, Andrew, Sydney, Co Antrim BT38 8FF (GB)
(74) Representative: McNally, Roisin
(86) International application number: PCT/GB1999/004104
(87) International publication number: WO 2000/034786

(56) References cited:
- BIOLOGICAL ABSTRACTS, Philadelphia PA USA; abstract no. PREV199800318350, abstract XP002133579 cited in the application & D. BRADFORD ET AL.: "Molecular biological methods to detect "Microthrix parvicella" and to determine its abundance in activated sludge " WATER SCIENCE AND TECHNOLOGY, vol. 37, no. 4-5, 1 February 1998 (1998-02-01), pages 37-45, Brisbane Australia

## Description

This invention relates to the field of detection of bacterial populations in waste waters, such as activated sludge, lagoon treatment ponds and other waste treatment methods in common use within industry. In particular the invention relates to detection of *Microthrix* species.

*Microthrix parvicella* is a bacterium difficult to detect *in situ* in mixed aqueous systems by conventional means. The present invention relates to a method to specifically detect and label *M. parvicella* in a variety of complex samples. By detecting and quantifying this organism, it will be possible to better control settling problems encountered in waste waters caused by this organism.

At present two methodologies are used to identify *M*. *parvicella* in mixed samples: by conventional taxonomic identification using keys developed by Eikelboom (1968) and others and by the use of specific fluorescently-labelled *in situ* hybridisation (FISH) DNA probes (designed from unique 16S rRNA DNA sequences). The former is used routinely within the industry and the latter is at present restricted to research studies (Erhart et al., 1997). Quantification of *M. parvicella* in pure culture is presently carried out by protein or dry weight determinations (c.f Slijkhuis et al. 1984). Various methods in the past have been applied to estimate numbers of filaments of *M. parvicella*, via direct counting methods (Bradford et al, 1998), but these are subjective and only give conservative estimates.

Key-type taxonomic identification is based on the combined use of the microscopic stains and morphological characteristics. As a result this is slow, and needs a significant degree of operator training and expense. Morphological identification is largely subjective, and does not take account of unusual growth forms of M parvicella that may exist. *In situ* DNA probes are highly specific, but are relatively expensive in the materials used. In addition, the time taken for each assay and cost is increased due to the need to permeablise the cells to enable introduction of the probe to the cell. The degree of fluorescence is variable according to the number of ribosomes present within the cell. During times of low nutrient availability, or other times where the cell is not growing, the number of ribosomes may be low, leading to a poor fluorescent signal. Additionally, the variable nature of waste water precludes the development of an essay method applicable to a wide range of samples on a routine basis. Enumeration from protein measurements and dry weight is only possible in pure cultures, is not applicable to the waste water industry, and can only provide an empirical estimation. Direct counts are dependant on correct initial diagnosis, and will fail to count filaments within flocs.

It is an object of the present invention to produce a method for diagnosis of populations of the bacteria *Microthrix parvicella* present in activated sludge or waste waters.

Further, it is also an object of the present invention to provide a method of quantification of *Microthrix parvicella* in instances where it is diagnosed.

Preferably, the method of detection and subsequent isolation to allow quantification, shall be mediated through the use of novel antibodies which have specificity to *M. parvicella.*

One suitable antibody is derivable from cell line MP5B106A deposited at the European Collection of Cell Culture on 30 November 1999 under Accession number 99113020.

Preferably, the specific novel antibodies will be detected through the use of an immunoassay or microscopic means.

The said antibodies may be conjugated to fluorescent agents, enzymes or similar molecules or indirectly labelled through the use of a secondary conjugated antibody, to enable the selective visualisation of cells of *M. parvicella*.

The antibodies may also be bound to an iron/polystyrene bead to allow immunomagnetic separation.

The current invention, further has the advantage that it provides a novel method of performing the difficult task of detecting *M. parvicella* in mixed aqueous systems, while at the same time providing considerable savings in costs, time and operator training required to perform the act by previous methods.

The present invention relates to several monoclonal antibodies showing specificity to candidatus *Microthrix parvicella.* Several hybrid Murine cell lines that secrete antibody specific to *Microthrix parvicella* are disclosed. These antibodies are useful in the detection and isolation of *Microthrix parvicella* from aqueous samples such as activated sludge.

*Microthrix parvicella* is a common causative bacterium of the waste water settling phenomenon known as filamentous bulking, especially those receiving municipal waste (Blackall et al., 1995, Eikelboom et al. 1998). It also causes extensive foaming and scumming of anaerobic digestors (Dillner Westlund et al. 1998). Several surveys conducted of waste water treatment plants in Western, Northern, Southern and Central Europe have indicated *M. parvicella* to be the dominant micro-organism (Eikelboom et al., 1998, Wanner et al. 1998). Current attempts to control this organism have only had limited success (Madoni and Davoli, 1993). Currently routine diagnosis can only be carried out in a qualitative and semi-quantitative manner using conventional microbiological taxonomic tools (Eikelboom, 1968), and is thus subject to ambiguous interpretations.

Therefore, it is an object of this application to provide novel monoclonal and polyclonal antibodies specific to candidatus *M. parvicella* suitable for unambiguous detection and enumeration.

The invention will be exemplified with reference to the accompanying drawings wherein
Figures 1a and 1b illustrates fluoresently labelled DNA probes based on 16S RNA.
Figure 2 shows immunofluorescence of *M. parvicella* RN1 using Rabbit polyclonal antibody.
Figure 3 illustrates immunofluorescence generated by a selection of antibodies.
Figure 4 shows a municipal waste sample probed with a cocktail of antibodies.
Figure 5 shows a municipal waste sample probed with a monoclonal antibody in phase contrast and fluorescent fields.
Figure 6 shows a municipal waste sample probed with a monoclonal antibody in phase contrast and fluorescent fields.
Figure 7 shows a municipal waste sample probed with a monoclonal antibody in phase contrast and fluorescent fields.
Figure 8 shows pharmaceutical waste probed with monoclonal antibody cocktail in phase contract and fluorescent fields.
Figure 9 shows polyclonal antibody differentiating between different growth states of *M. parvicella*.

A novel monoclonal antibody according to the present invention is produced as follows. That is, the anti-*M. parvicella* antibody is produced by immunising a BALB/c mouse with the Gram-positive heterotroph *M. parvicella* strain RN1 isolated from a conventional waste water treatment facility (Rossetti et al., 1997), forming fused cells (hybridomas) between antibody-forming spleen cells obtained from the animal and mouse myeloma cells (P3x63Ag8-653 (NS-0/1) cell line), subsequently proliferating said fused cells, selecting cells forming an antibody showing specificity to *M. parvicella* from the proliferated fused cells, and culturing antibody-forming cells producing the same.

An anti-*M. parvicella* antibody would be useful in the quantitative and qualitative assessment of populations of *M.parvicella* present in activated sludge or other waste waters, either by immunofluorescent microscopy or by some other immunoassay known to the art, or as a reagent to be used in extracting other strains of *M. parvicella* from mixed microbial communities.

Polyclonal antibodies specific to *M. parvicella* are prepared by immunising a rabbit to *M. parvicella* RN1. After a suitable number of immunisations, the animal is bled out, and serum containing polyclonal antibody obtained.

For detection of an antibody, an enzyme immunoassay is employed. The production of monoclonal antibody to *M. parvicella* is performed by maintaining and growing the above antibody-forming clones in a RPMI medium (ICN-Flow laboratories, UK)

The present invention offers numerous advantages over present techniques:
1. Identification of *M. parvicella* is unambiguous, due to the high specificity of the antibodies used.
2. No cell pre-treatment step is required, cutting a significant amount of time and cost from the total assay. This also allows the method to be applied to flow cytometric analysis, allowing on-line analysis.
3. The signal strength is superior and more even over the cells than that obtained by using 16S rRNA probes.
4. The assay is simple, requiring few steps and only minimal operator training. Most components of the assay are inexpensive and easily sourced.

### A. Preparation of monoclonal antibody

*Microthrix parvicella* RN1 was cultivated on a complex agar medium (R2AM: per gram double distilled water; Yeast Extract, 0.5g, Protease Peptone, 0.5g Casamino Acids, 0.5g, Soluble Starch, 0.5g, MgSO₄.7H₂O, 0.10g, KH₂PO₄, 0.3g CaCl₂.2H₂O, 0.05g, NaEDTA, 50mg, Na₂MoO₄, 20mg, NMS trace element solution (Atlas, 1996) 1ml, vitamin supplement (Eikelboom, 1968), 1ml, Glucose, 0.5g, Pyruvate, 0.5g) at an incubation temperature of 15-20°C for 3-4 weeks, or until colonies are visible.

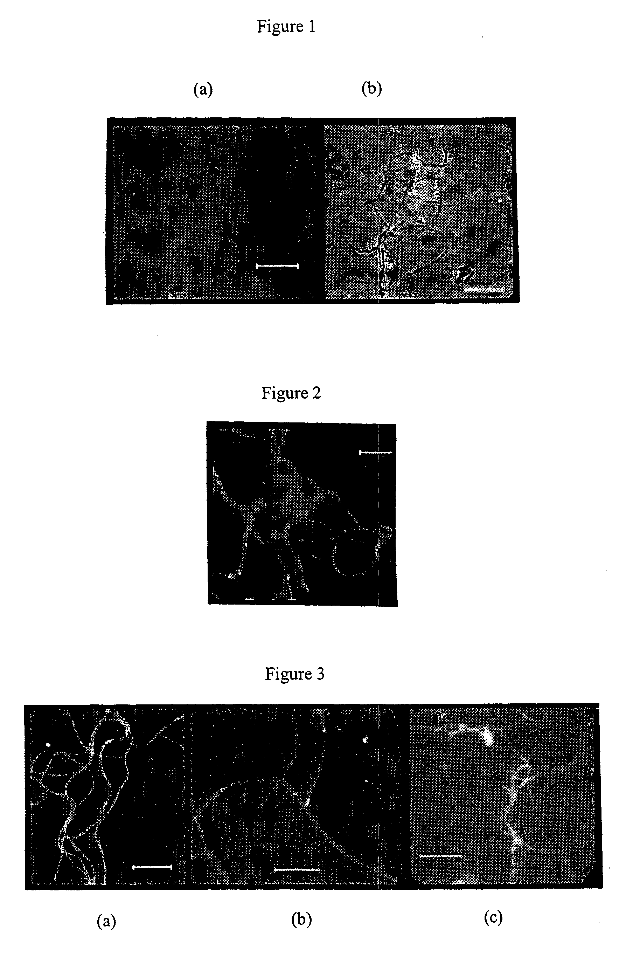

The microbial identity of *Microthrix* parvicella RN1 was confirmed by partially sequencing the 16S rRNA gene and by fluorescent in situ hyrbidisation (by the method of Erhart et al, 1997) (figure 1a) using species-specific 16S rRNA oligonucleotides. Figure 1b shows the appearance of the bacterium under ordinary (white lite, phase contrast) conditions.

For production of all antibodies, bacterial biomass was obtained by washing 30 day old cell colonies from R2AM agar with physiologically buffered saline (PBS).

Polyclonal antibody was obtained by immunizing a rabbit subcutaneously with 0.8 mls of a suspension (delivered in 4 locations in 0.2 ml amounts) of *M. parvicella* RN1 in PBS (optical density of approximately 0.6 at 650 nm) every 10 days until immunofluorescence of *M. parvicella* RN1 on slides could be determined using sampled serum (in this case, after 3 such immunisations). At this point, the host animal, a Rabbit, was bled out, and the serum obtained according to the art. Serum was used in immunofluorescence using a suitable dilution in PBS. Figure 2 shows immunofluorescence of *M. parvicella* RN1 using a 1:1000 dilution of Rabbit polyclonal antibody.

Cell colonies were then harvested/washed with a physiological saline solution (PBS) and suspended in PBS at pH 7.3 to give an absorbance at 650 nm of 0.2000. 0.2 ml of the obtained suspension was injected into the peritoneal cavity of a BALB/c mouse, and 36 days later 0.2 ml of the same suspension was injected in the same manner. The mouse was killed on the 4th day following the final immunisation to extract the spleen, and the spleen cells were suspended in the RPMI 1640 medium (ICN-Flow Laboratories, UK). Standard cell fusion was then carried out in a manner known to the art (Lutton et al., 1991), and single cell clones obtained. These clones were then cultivated in RPMI 1640 medium, and screened for IgG production by standard means. Positive hybridomas were selected and further cultured and screened for antibody specific to *M. parvicella* by the immunofluorescence method as follows.

Following this, clones were recloned by limiting dilution, and rescreened for specificity. The following cell lines were obtained: MP5B106A, MP4B5, MP4C3, MP2C11 and MP3E12. The immunofluorescence generated by a selection of antibodies (MP5B106A, MP4B5, and MP2C11, chosen as representative of general appearance) when reacted with *M. parvicella* RN1 and a fluorescent anti-Mouse IgG antibody as shown in figures 3a-3c.

The specificity of the presumptive monoclonal antibodies to *Microthrix parvicella* bacteria was determined by immunofluorescence microscopy and by a colour reaction according to an enzyme antibody assay. Immunofluorescence microscopy was performed as described by Ramage et al. (1998): monoclonal antibody was bound to formaldehyde-killed cells fixed to a microscope slide through a series of incubations. A secondary anti-mouse goat antibody, labelled with either TRITC or FITC (Biorad, USA), was bound to this antibody following a further series of incubations. UV-microscopy, employing a suitable filter, allowing visualisation of labelled cells. Non-labelled cells did not fluoresce under those conditions.

The specificity of the monoclonal and polyclonal antibodies with respect to other filamentous and non-filamentous bacteria commonly found in wastewater treatment plants are examined. Cultures of *Escherichia coli* (NCIMB Type strain 11943), *Haliscomenobacter* hydrossis (ATCC 27775), *Sphaerotilus natans* (NCIMB 11196), *Thiothrix* spp. isolate (provided by R. Brigmon, Westinghouse Savannah River Company, South Carolina, US, isolated from Orange Springs, Florida), Gordonae amarae (nocardia arnarae NCIMB 11222), *Propionibacterium* acnes (Type 1 and 2), Rhodococcus MJL100 (NCIMB 12038) and *Lactobacillus* plantarum were examined. None showed any significant cross-reactivity following immunofluorescence microscopy.

### B. Detection of M. parvicella in samples

The assay used follows that described by Ramage et al (1998) :
1. Air dry a 30 µl sample on a Teflon coated multiwell slide (ICN-Flow Laboratories, UK).
2. Two methods can equally be used to fix the cells on the slide, Either fix in 4% Formaldehyde (in physiologically buffered aline (PBS)) at 4°C, followed by dehydration in ice cold absolute alcohol for 10 minutes. Or fix air-dried smears in ice cold methanol for 10 minutes. In both cases, air-dry the slide prior to storage or use.
3. Apply 30 µl of antibody solution to the air dried smear (at the appropriate dilution) and incubate at 37°C for 45 minutes.
4. Wash off antibody, wash in PBS for 30 minutes.
5. Apply secondary fluoroscently-labelled (e.g FITC) anti-mouse goat antibody (Biorad, USA), and incubate as before for 45 minutes. Wash again for 30 minutes.
6. Mount slide and view with a epifluorescence microscope, or similar. Filamentous cells of *M. parvicella* will fluoresce according to the fluorescent label and filter used.

Other methods may vary in the type of labelled secondary antibody, or, alternatively, the anti-*M.* *parvicella* antibody may be directly labelled with a suitable chemical tag according to the methods known to the art. The labelled antibody may also be applied directly to a sample for flow cytometric analysis (i.e computerised analysis of fluorescent particles passing through a laser).

Presumptive *Microthrix* parvicella filaments were detected in a range of samples from petrochemical waste, municipal waste water plants and fisheries waste using the aforementioned immunofluorescence. No fluorescent filaments were detected in a range of samples (other municipal waste water treatment plants, other petrochemical plants, pharmaceutical plant waste) previously determined to be free of *M. parvicella.*

The following figures illustrate the detection of natural populations of *M. parvicella* populations in wastewaters by immunofluorescene microscopy.

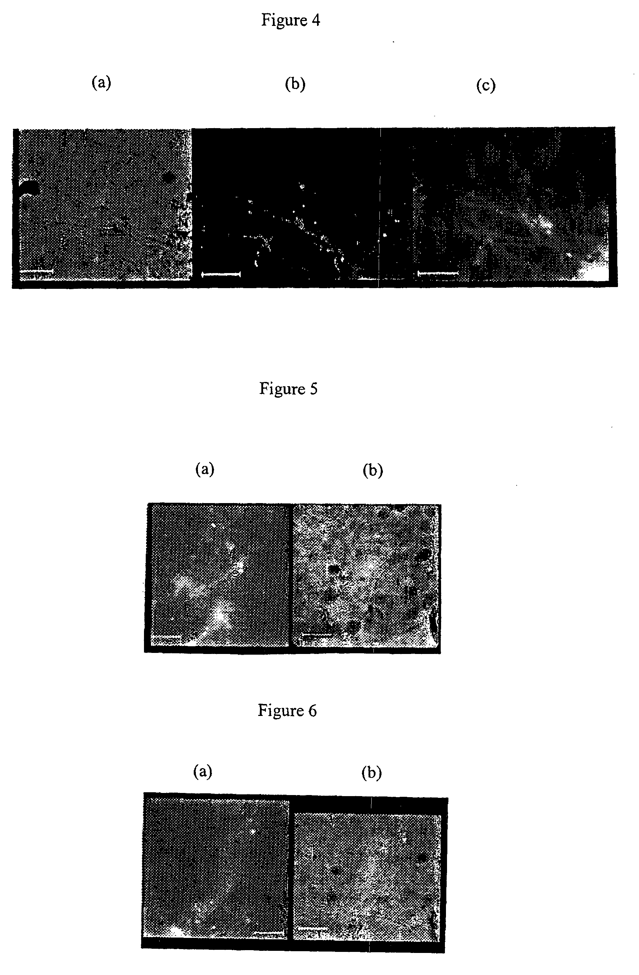

Figures 4a to 4c show a municipal waste sample dual probed with an FITC-labelled cocktail of the described monoclonal antibodies and TRITC-labelled polyclonal antibody as described. Identical fields of view under both phase contrast and UV-excitation are shown (the UV-excitation image showing fluorescent filaments identified as *M. parvicella*). In both cases, the same filaments are seen fluorescing.

Figures 5a, 5b, 6a, 6b, 7a and 7b shows a municipal waste sample probed with FITC-labelled monoclonal antibody (MP2C11, MP4B5 and MP5B106A respectively); both the phase contrast and fluorescing fields of view are shown. Fluorescing filaments were identified as *M. parvicella*.

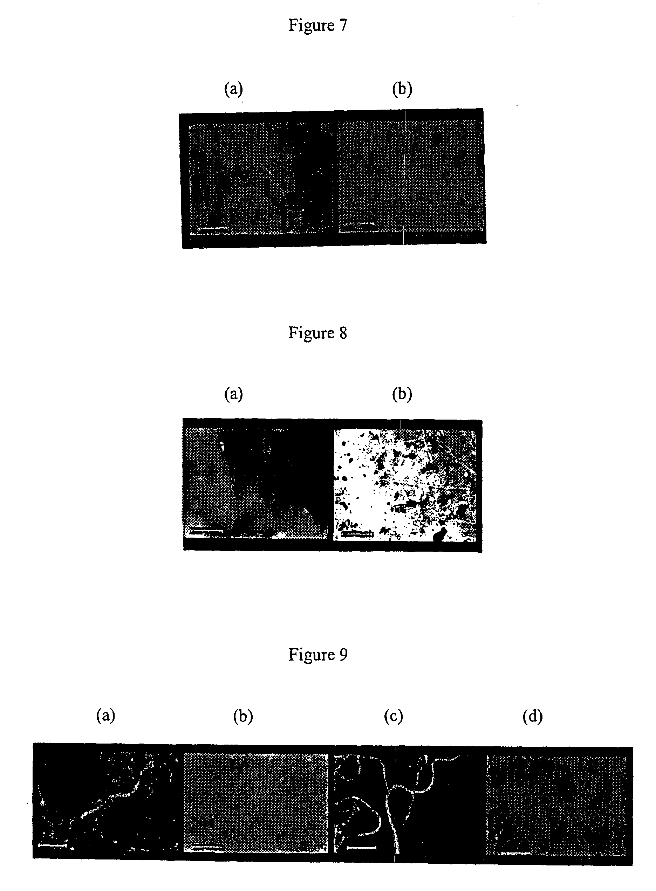

Figures 8a and 8b show pharmaceutical waste previously determined not to contain *M. parvicella* probed with the FITC-labelled monoclonal antibody cocktail described previously. Non-*Microthrix* filaments show no fluorescence.

Figures 9a to 9d show the ability of the polyclonal antibody to differentiate different growth states of *M. parvicella* in situ. Levels of fluorescence differ in two municipal waste samples differentiated only by their relative oxygen content. This indicates that the presentation of some of the epitopes is affected by the growth state of the bacterium.

Fluorescently labelling the bacteria greatly improves the applicability of digital image analysis, using commercially available software such as PC Image (Foster Findlay Associates) or similar, to enable enumeration of specific sub-populations.

The fluorescently labelled antibody may also be applied directly to a sample for flow cytometric analysis (i.e. computerised analysis of fluorescent particles passing through a laser) (Leiserson, 1985).

Other applicable microscopic methods may vary in the type of labelled secondary antibody, or, alternatively, the anti-*M. parvicella* antibody may be directly labelled with a suitable chemical tag according to methods known to the art. Such variations include:
i) Immunogold or immunosilver labelling, suitable for ordinary light microscopy.
ii) Horseradish peroxidase and alkaline phosphase with a solid substrate can also be used to give a coloured marker when bound to a secondary antibody.
iii) Radiolabelled secondary antibodies may also be used, and cells visualised by micro-autoradiography.

### C. Immunomagnetic separation

Another application is incorporation into a commercially available immunomagnetic separation. In this case, the antibody is bound to a plastic bead, with an iron core. Target cells bind onto the attached antibody. The beads, with attached cells can then be removed from the sample by use of a magnet. This method can be used for the removal of *M. parvicella* from a sample for research purposes.

Target cells or extract may be concentrated onto a suitable membrane (e.g. nitro-cellulose). Antibody is then applied, incubated, excess washed, followed by a secondary antibody, labelled with either horseradish peroxidase or alkaline phosphatase. In the presence of a suitable solid substrate, and following a prescribed incubation step, target cells will be colormetrically labelled and quantified using a suitable colorimeter.

### References

**Atlas, RM** (1996). Handbook of Media for Environmental Microbiology. CRC Press. London
**Blackall, L.L., Seviour, E. M., Cunningham, M. A., Seviour, R. J., and Hugenholtz, P.** (1995). Microthrix parvicella is a novel, deep branching member of the actinomycetes subphylum. *Systematic and Applied Microbiology* **17,** 513-518.
**Bradford, D., Christensson, C., Jakab, N. and Blackall, L.L** (1998). Molecular biological methods to detect "Microthrix parvicella" and to determine its abundance in activated sludge. *Water Science and Technology* **37,** 37-45.
**Dillner-Westlund, A., Hagland, E., and Rothman, M.** (1998). Bulking and foaming caused by Microthrix parvicella at three large sewage treatment plants in the Stockholm area. *Water Science and Technology* **34**, 281-287.
**Eikelboom, D.H., Andreadakis, A. and Andreasen, K** (1998). Survey of filamentous populations in nutrient removal plants in four European countries. *Water Science and Technology* **37**, 281-289.
**Eikelboom, D. H.** (1968). Filamentous organisms observed in activated sludge. *Water Research* **9**, 365-388
**Erhart, R., Bradford, D., Seviour, R. J., Amann, R., and Blackall, L. L.** (1997). Development and use of fluorescent in situ hybridisation probes for the detection and identification of "Microthrix parvicella" in activated sludge. *Systematic and Applied Microbiology* **20,** 310-318.
**Hill, E., and Matsen, J.** (1983). Enzyme-linked immunoabsorbent assay and radioimmunoassay in the serologic diagnosis of infectious diseases. *Journal of Infectious Diseases* **147**:258.
**Lutton, D.A., Patrick, S., Crockard, A.D., Stewart, L.D., Larkin, M.J., Dermott, E., and McNeill, T.A** (1991). Flow cytometric analysis of within-strain variation in polysaccharide expression by Bacteroides fragilis by use of murine monoclonal antibodies. *Journal of Medical Microbiology* **35,** 229-237.
**Madoni, P., and Davoli, D.** (1993). Control of Microthrix-parvicella growth in activated-sludge. *Fems Microbiology Ecology* **12,** 277-284.
**Ramage, G., Patrick, S., and Houston, S** (1998). Combined fluorsecent in situ hybridisation and immunolabelling of Bacteroides fragilis. *Journal of Immunological* Methods **212,** 139-147.
**Rossetti, S., Christensson, C., Blackall, L. L., and Tandoi, V.** (1997). Phenotypic and phylogenetic description of an Italian isolate of "Microthrix parvicella". *Journal of Applied Microbiology* **82,** 405-410.
**Slijkhuis, H., Van Groenestijn, J. and Kylstra, D.J.** (1984). Microthrix parvicella, a filamentous bacterium from activated sludge: Growth on Tween 80 as carbon and energy source. *Journal of General Microbiology* **130,** 2035-2042.
**Tandoi, V., Rossetti, S., Blackall, L. L. and Majone, M.** (1998). Some physiological properties of an Italian isolate of "Microthrix parvicella". *Water Science and Technology* **37,** 1-8.
**Wanner, J., Ruzicková, I., Jetmarová, P., Krhutkvoá, O. and Paraniaková J.** (1998). A national survey of activated sludge separation problems in the Czech Republic: Filaments, floc characteristics and activated sludge metabolic properties. *Water Science and Technology* **37.**

## Claims

1. A method of detecting Microthrix *parvicella*, the method comprising the step of reacting *M. parvicella* with antibodies which have specificity to *M. parvicella*.

2. A method as claimed in claim 1 which comprises an immuno assay and/or microscopic means.

3. A method as claimed in any of the preceding claims wherein the antibodies are conjugated to fluorescent agents or enzymes or indirectly labelled through the use of a secondary conjugated antibody to enable selective visualisation of cells of *M. parvicella.*

4. A method as claimed in any of the preceding claims which further includes a step to isolate the *M. parvicella*.

5. A method as claimed in any of the preceding claims wherein the antibodies are bound to iron/polystyrene bead(s) to allow immunomagnetic separation.

6. A method as claimed in any of the preceding claims wherein *M. parvicella* is detected in an aqueous system.

7. A method as claimed in any of the preceding claims wherein the antibody is a monoclonal antibody.

8. A method as claimed in any of the preceding claims wherein the antibody consists of at least two monoclonal antibodies.

9. A method as claimed in any of the preceding claims wherein the antibody is derivable from cell line MP5B106A deposited at The European Collection of Cell Cultures on 30 November 1999 under Accession number 99113020.

10. A method for detecting *Microthrix parvicella* according to claim 1 in a sample, the method comprising;
applying antibodies which specifically bind *Microthrix parvicella* to the sample,
detecting the presence or absence of binding of the antibodies in the sample wherein binding of the antibodies is indicative of the presence of *Microthrix parvicella*.

11. Cell line MP5B106A deposited at The European Collection of Cell Cultures on 30 November 1999 under accession number 99113020.

12. Antibodies produced from the cell line as claimed in claim 11.

13. Antibodies as claimed in claim 12 for use in a method as claimed in any of claims 1 to 9.

14. A cocktail of antibodies consisting of at least two monoclonal antibodies and including at least one monoclonal antibody produced from the cell line as claimed in claim 11 for use in a method as claimed in any of claims 1 to 9.

## Patentansprüche

1. Ein Verfahren zum Erkennen von *Microthrix parvicella*, wobei das Verfahren den Schritt beinhaltet, *M. parvicella* mit Antikörpern, die eine Spezifität für *M. parvicella* aufweisen, reagieren zu lassen.

2. Verfahren gemäß Anspruch 1, das einen Immunoassay und/oder mikroskopische Mittel beinhaltet.

3. Verfahren gemäß einem der vorhergehenden Ansprüche, wobei die Antikörper mit Fluoreszenzstoffen oder Enzymen konjugiert oder indirekt durch die Verwendung eines sekundären konjugierten Antikörpers markiert sind, um eine selektive Sichtbarmachung der Zellen von *M. parvicella* zu ermöglichen.

4. Verfahren gemäß einem der vorhergehenden Ansprüche, das ferner einen Schritt umfasst, um *M. parvicella* zu isolieren.

5. Verfahren gemäß einem der vorhergehenden Ansprüche, wobei die Antikörper an (eine) Eisen-/Polystyrol-Perle(n) gebunden sind, um eine immunmagnetische Trennung zu erlauben.

6. Verfahren gemäß einem der vorhergehenden Ansprüche, wobei *M. parvicella* in einem wässrigen System erkannt wird.

7. Verfahren gemäß einem der vorhergehenden Ansprüche, wobei der Antikörper ein monoklonaler Antikörper ist.

8. Verfahren gemäß einem der vorhergehenden Ansprüche, wobei der Antikörper aus mindestens zwei monoklonalen Antikörpern besteht.

9. Verfahren gemäß einem der vorhergehenden Ansprüche, wobei der Antikörper von der Zell-Linie MP5B106A, die an der European Collection of Cell Cultures (Europäische Zellkultursammlung) am 30. November 1999 unter der Zugangsnummer 99113020 hinterlegt worden ist, herleitbar ist.

10. Verfahren zum Erkennen von *Microthrix parvicella* gemäß Anspruch 1 in einer Probe, wobei das Verfahren Folgendes beinhaltet:
Anwenden von Antikörpern, die *Microthrix parvicella* spezifisch an die Probe binden, Erkennen der Anwesenheit oder Abwesenheit von Bindung der Antikörper in der Probe, wobei die Bindung der Antikörper auf die Anwesenheit von *Microthrix parvicella* hinweist.

11. Zell-Linie MP5B106A, hinterlegt an der European Collection of Cell Cultures am 30. November 1999 unter der Zugangsnummer 99113020.

12. Antikörper, produziert von der Zell-Linie gemäß Anspruch 11.

13. Antikörper gemäß Anspruch 12 zur Verwendung in einem Verfahren gemäß einem der Ansprüche 1 bis 9.

14. Ein Cocktail von Antikörpern, der aus mindestens zwei monoklonalen Antikörpern besteht und mindestens einen monoklonalen Antikörper umfasst, der von der Zell-Linie gemäß Anspruch 11 zur Verwendung in einem Verfahren gemäß einem der Ansprüche 1 bis 9 produziert worden ist.

## Revendications

1. Un procédé de détection de *Microthrix parvicella*, le procédé comportant l'étape de faire réagir *M. parvicella* avec des anticorps qui ont une spécificité par rapport à *M*. *parvicella*.

2. Un procédé tel que revendiqué dans la revendication 1, lequel comporte un immunoessai et/ou un moyen microscopique.

3. Un procédé tel que revendiqué dans n'importe lesquelles des revendications précédentes, dans lequel les anticorps sont conjugués à des agents fluorescents ou des enzymes ou sont indirectement marqués grâce à l'utilisation d'un anticorps conjugué secondaire pour permettre la visualisation sélective des cellules de *M*. *parvicella*.

4. Un procédé tel que revendiqué dans n'importe lesquelles des revendications précédentes, lequel comprend de plus une étape pour isoler *M. parvicella*.

5. Un procédé tel que revendiqué dans n'importe lesquelles des revendications précédentes, dans lequel les anticorps sont liés à une ou des bille(s) de fer/de polystyrène pour permettre la séparation immunomagnétique.

6. Un procédé tel que revendiqué dans n'importe lesquelles des revendications précédentes, dans lequel *M. parvicella* est détecté dans un système aqueux.

7. Un procédé tel que revendiqué dans n'importe lesquelles des revendications précédentes, dans lequel l'anticorps est un anticorps monoclonal.

8. Un procédé tel que revendiqué dans n'importe lesquelles des revendications précédentes, dans lequel l'anticorps consiste en au moins deux anticorps monoclonaux.

9. Un procédé tel que revendiqué dans n'importe lesquelles des revendications précédentes, dans lequel l'anticorps peut être dérivé de la lignée cellulaire MP5B106A déposée à The European Collection of Cell Cultures le 30 novembre 1999 sous le numéro d'accès 99113020.

10. Un procédé destiné à détecter *Microthrix parvicella* selon la revendication 1 dans un échantillon, le procédé comportant ;
appliquer des anticorps qui lient de façon spécifique *Microthrix parvicella* à l'échantillon,
détecter la présence ou l'absence de liaison des anticorps dans l'échantillon dans lequel la liaison des anticorps est indicative de la présence de *Microthrix parvicella*.

11. La lignée cellulaire MP5B106A déposée à The European Collection of Cell Cultures le 30 novembre 1999 sous le numéro d'accès 99113020.

12. Des anticorps produits à partir de la lignée cellulaire telle que revendiquée dans la revendication 11.

13. Les anticorps tels que revendiqués dans la revendication 12 destinés à être utilisés dans un procédé tel que revendiqué dans n'importe lesquelles des revendications 1 à 9.

14. Un cocktail d'anticorps consistant en au moins deux anticorps monoclonaux et comprenant au moins un anticorps monoclonal produit à partir de la lignée cellulaire telle que revendiquée dans la revendication 11 destiné à être utilisé dans un procédé tel que revendiqué dans n'importe lesquelles des revendications 1 à 9.
